# FASCICULE DE BREVET EUROPEEN

(11) **EP 3 182 382 B1**
(45) Date de publication et mention de la délivrance du brevet: **28.07.2021**
(21) Numéro de dépôt: 16199177.3
(22) Date de dépôt: 16.11.2016
(51) Int. Cl.: G07C 1/24, A63B 71/06

(54) **SYSTÈME DE MESURE AVEC CORRECTION DU TEMPS D'ARRIVÉE D'UN ATHLÈTE DANS UNE COURSE**
MESSSYSTEM MIT KORREKTUR DER ANKUNFTSZEIT EINES ATHLETEN BEI EINEM RENNEN
MEASUREMENT SYSTEM WITH CORRECTION OF THE ARRIVAL TIME OF AN ATHLETE IN A RACE

(30) Priorité: 18.12.2015 EP 15201308
(43) Date de publication de la demande: 21.06.2017
(73) Titulaire: Swiss Timing Ltd., 2606 Corgémont (CH)
(72) Inventeur: Galli, Reto, 3053 Münchenbuchsee (CH); Kollega, Alexander, 04275 Leipzig (DE); Massé, Fabien, 1004 Lausanne (CH)
(74) Mandataire: Giraud, Eric

(56) Documents cités:
- EP-A1- 1 369 146
- CH-A2- 707 401
- US-A1- 2013 044 043
- US-A1- 2015 116 497

## Description

### Domaine de l'invention

L'invention concerne un système de mesure avec correction du temps d'arrivée d'un athlète dans une course à pieds au moyen d'un module à transpondeur adapté à l'athlète et porté de préférence sur une partie haute du corps de l'athlète durant la course.

L'invention concerne également un procédé de mesure avec correction du temps d'arrivée d'un athlète dans une course à pieds pour la mise en action du système de mesure.

### Arrière-plan de l'invention

Lors d'une compétition sportive comme une course de sprint en athlétisme, chaque athlète part depuis un starting-block. Chaque athlète est souvent équipé d'un module à transpondeur, qui est intégré dans le dossard à numéro. Le chronométrage ou le temps de course non officiel de l'athlète est pris depuis la lecture du module à transpondeur, qui apparaît lorsque ledit module à transpondeur passe la ligne d'arrivée. Après cela, le temps officiel est jugé suite à l'image photofinish prise en tenant compte des règles IAAF 165.2.

Il est à noter que ce temps doit être pris au moment, où n'importe quelle partie du torse, atteint le plan vertical à proximité du bord de la ligne d'arrivée. Ce temps officiel est normalement disponible environ entre 10 et 20 s après le temps non officiel, ce qui constitue une longue période pour des spectateurs visionnant ladite course.

Si un athlète passe la ligne d'arrivée en étant dans une position droite, le module à transpondeur est dans une position, qui correspond tout à fait à la position pour juger le temps sur l'image photofinish. Ainsi, l'erreur entre le temps non officiel du module à transpondeur et le temps officiel tiré de l'image photofinish diffère seulement en fonction de l'imprécision de la détection du module à transpondeur. Cependant, beaucoup d'athlètes se plient vers l'avant, lorsqu'ils passent la ligne d'arrivée pour obtenir un avantage dans le jugement par image photofinish. Dans ce cas, l'athlète est jugé sur les épaules vers l'avant avec l'image photofinish, alors que le module à transpondeur passe la ligne d'arrivée plus de 10 cm derrière ce point d'arrivée. Ainsi, la différence entre les temps non officiel du module à transpondeur et officiel peut être assez grande. De plus, dans le cas d'une arrivée serrée de plusieurs athlètes, le classement de chacun peut aussi être erroné.

Selon l'état de la technique, et en fonction de la différence de temps susmentionnée entre le temps du transpondeur et le temps de l'image photofinish, le classement non officiel est publié uniquement quand la différence du temps du module à transpondeur de deux athlètes est plus grande que 0.1 s. Si la différence est plus faible, aucun classement n'est montré aux spectateurs, jusqu'à ce que les temps officiels de l'image photofinish soient disponibles.

On peut citer la demande de brevet CH 707 401 A2, qui décrit un procédé et un système pour la mesure d'un temps dans une compétition sportive avec au moins un module à transpondeur personnalisé. Le module à transpondeur peut être placé sur le compétiteur ou sur un objet mobile l'accompagnant par exemple un vélo ou un ski durant la course. Le module peut être activé soit au départ de la course, soit dans des positions intermédiaires du parcours de course ou à l'arrivée. Une variation de mouvement est détectée par un capteur de mouvement dans le module. Les mesures du mouvement peuvent être transmises du module à une unité à décodeur pour contrôler un temps de course. Cependant il n'est pas défini une correction d'un temps d'arrivée en fonction des mesures effectuées par le capteur de mouvement.

### Résumé de l'invention

L'invention a donc pour but de pallier les inconvénients de l'état de la technique susmentionné en proposant un système de mesure avec correction du temps d'arrivée d'un athlète dans une course à pieds au moyen d'un module à transpondeur adapté à l'athlète et porté sur une partie haute du corps de l'athlète durant la course.

A cet effet, l'invention concerne un système de mesure avec correction du temps d'arrivée d'un athlète dans une course, qui comprend les caractéristiques définies dans la revendication indépendante 1.

Des formes d'exécution particulières du système de mesure avec correction du temps d'arrivée d'un athlète dans une course sur piste sont définies dans les revendications dépendantes 2 à 6.

Un avantage du système de mesure avec correction du temps d'arrivée réside dans le fait qu'avec le module à transpondeur placé sur une partie haute du corps de l'athlète, il est possible de corriger le temps d'arrivée d'un athlète suite au passage du module à transpondeur sur la ligne d'arrivée. Le module à transpondeur comprend au moins un capteur de mouvement capable de fournir une information sur un angle d'inclinaison de la partie haute du corps de l'athlète lors du passage de la ligne d'arrivée. Avec l'information fournie par le module à transpondeur sur l'instant de passage de la ligne d'arrivée et l'angle d'inclinaison déterminé, cela permet d'estimer le temps d'arrivée du module à transpondeur. Une correction du temps d'arrivée non officiel sur la base du passage du module à transpondeur sur la ligne d'arrivée peut ainsi être calculée.

Le module à transpondeur comprend au moins un capteur de mouvement pour mesurer l'angle l'inclinaison de la partie haute du corps de l'athlète. Ce capteur de mouvement est un capteur de mouvement inertiel. Il est un gyroscope relié à une unité de traitement capable d'effectuer des calculs sur la base des mesures du gyroscope pour transmettre l'information d'angle d'inclinaison au passage de la ligne d'arrivée. Le gyroscope doit en principe être calibré initialement pour pouvoir déterminer une différence d'orientation, c'est-à-dire un angle d'inclinaison par rapport à une direction verticale ou horizontale.

Le module à transpondeur est placé sur une partie haute du corps de l'athlète en particulier dans le dossard à numéro. Une fois calibré et activé, le gyroscope détermine l'inclinaison de l'athlète au moment du passage de la ligne d'arrivée pour pouvoir corriger le temps d'arrivée non officiel déterminé et fourni par le module à transpondeur par rapport au temps officiel déterminé avec l'image photofinish. Grâce au signal de données transmis par le module à transpondeur, un classement correct non officiel des athlètes au terme de leur course peut être immédiatement fourni à l'arrivée d'une course sur piste, par exemple une course de sprint.

A cet effet, l'invention concerne aussi un procédé de mesure avec correction du temps d'arrivée d'un athlète dans une course à pieds pour la mise en action du système de mesure, et qui comprend les caractéristiques définies dans la revendication indépendante 7.

Des étapes particulières du procédé de mesure sont définies dans les revendications dépendantes 8 à 10.

### Brève description des dessins

Les buts, avantages et caractéristiques du système de mesure avec correction du temps d'arrivée d'un athlète dans une course à pieds, et le procédé de mise en action du système de mesure apparaîtront mieux dans la description suivante d'au moins une forme d'exécution non limitative illustrée par les dessins sur lesquels :
la figure 1 représente schématiquement les principaux éléments d'un système de mesure avec correction du temps d'arrivée d'un athlète dans une course à pieds selon l'invention,
les figures 2a à 2c représentent de manière schématique un athlète, qui est muni d'un module à transpondeur montrant une vue de son passage d'une ligne d'arrivée en position droite et deux vues avec la partie haute du corps pliée au passage de la ligne d'arrivée, et
la figure 3 représente de manière schématique un athlète, qui est muni d'un module à transpondeur en position de départ sur un starting-block.

### Description détaillée de l'invention

Dans la description suivante, tous les éléments du système de mesure avec correction du temps d'arrivée d'un athlète dans une course à pieds, qui sont bien connus de l'homme du métier dans ce domaine technique, ne seront relatés que de manière simplifiée. La course à pieds peut être effectuée sur piste ou sur route ou en forêt ou en montagne.

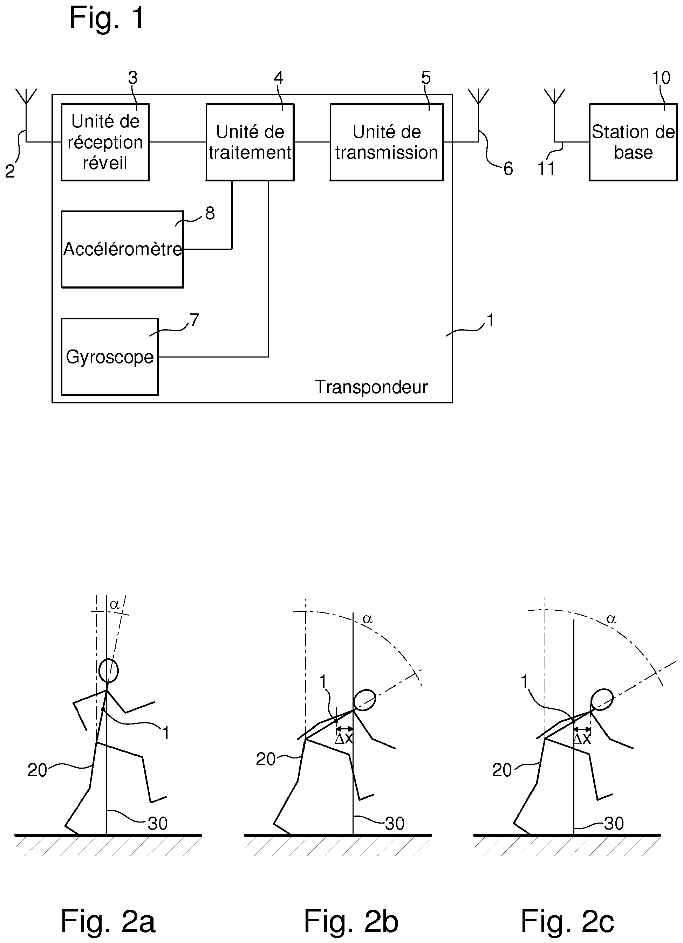

La figure 1 représente schématiquement les principaux éléments, qui composent un système de mesure avec correction du temps d'arrivée d'un athlète dans une course sur piste ou sur route. Pour ce faire, le système comprend un ou plusieurs modules à transpondeur 1 et au moins une station de base 10 pour la communication de données et/ou de mesures et/ou de commandes entre les modules à transpondeur 1 et la station de base 10. Chaque module à transpondeur 1 pour la compétition est disposé sur une partie du corps d'un athlète, par exemple dans un dossard à numéro, et est donc personnalisé ou adapté à l'athlète, qui le porte.

Le module à transpondeur 1 est disposé sur une partie haute du corps de l'athlète, tel que le thorax. Comme expliqué ci-après, ledit module à transpondeur est disposé au niveau du centre gravité, tel que le thorax. Ceci permet de détecter la rotation du haut du corps de l'athlète notamment au moment de passer la ligne d'arrivée pour corriger le temps de course non officiel de l'athlète.

De préférence au niveau de la ligne d'arrivée, il est prévu une boucle antenne au sol ou une antenne de côté alignée avec la ligne d'arrivée pour pouvoir transmettre un signal de champ magnétique. Ce signal de champ magnétique peut être stationnaire ou avec modulation de synchronisation par période déterminée. Le module à transpondeur 1 de chaque athlète en course peut détecter le passage de la ligne d'arrivée en mesurant une ou plusieurs intensités du champ magnétique à proximité de l'antenne d'arrivée. Une prédiction du temps d'arrivée par le module à transpondeur 1 peut aussi être déterminée sur cette base. Une mesure du temps peut aussi être effectuée dans le module à transpondeur 1 ou par transmission de signaux de mesure d'intensités du champ capté à la station de base 10. Il est également possible que le module à transpondeur 1 soit réveillé et/ou synchronisé par une autre antenne au départ ou en différents points de la piste de course.

Le module à transpondeur 1 peut être du type actif avec une batterie ou cellule solaire ou autre source d'énergie intégrée dans le module ou du type passif en étant alimenté par la réception d'un signal d'interrogation ou de réveil traditionnel. Cependant avec l'utilisation d'un ou plusieurs capteurs de mouvement dans le module à transpondeur, il est nécessaire d'avoir un module à transpondeur du type actif.

Le module à transpondeur 1 comprend une unité de réception sans fil de signaux 3 pour recevoir par une antenne 2 des signaux de données ou commandes provenant d'une station de base 10 ou d'un émetteur disposé dans un starting-block du système de mesure ou le long d'un tracé ou de la piste de course ou également au niveau de l'arrivée de la course. De préférence, les signaux reçus par l'antenne 2 liée à l'unité de réception 3 sont des signaux permettant le réveil ou la synchronisation du module à transpondeur 1, qui peut être dans un état de repos avant la réception de tels signaux. Ces signaux de synchronisation sont générés, comme indiqué ci-dessus, par la station de base 10 ou par un émetteur du starting-block ou le long de la piste de course, après le signalement de préparation de départ d'une course d'athlétisme notamment ou directement au moment du coup de pistolet de départ.

Il est à noter que le module à transpondeur 1 peut aussi ne pas comprendre d'unité de réception 3, afin de n'effectuer que des mesures par au moins un capteur de mouvement et transmettre l'information à la station de base pour la correction d'un temps d'arrivée.

Le module à transpondeur 1 comprend encore une unité de traitement 4, qui peut être une machine d'état, un processeur ou un microcontrôleur, pour la gestion de toutes les données ou commandes ou mesures à recevoir ou à transmettre. Une synchronisation de ladite unité de traitement peut également être opérée dès le départ de course, si elle comporte une base de temps pour permettre la détermination d'un temps de course. La synchronisation du module à transpondeur 1 peut également être effectuée en différents points de la course ou à proximité de la ligne d'arrivée. L'unité de traitement 4 reçoit les données ou commandes mises en forme dans l'unité de réception 3 de manière également à réveiller tous les composants, qui composent le module à transpondeur 1. L'unité de traitement 4 est encore reliée à une unité de transmission de signaux 5 par une antenne 6 à destination de la station de base 10. La station de base 10 peut être un système de chronométrage de la course et comprend une antenne 11 de transmission ou réception de signaux.

Le module à transpondeur 1 comprend encore au moins un capteur de mouvement 7 lié à l'unité de traitement 4 pour fournir des signaux de mesure en continu ou par intermittence à l'unité de traitement 4 une fois que le module à transpondeur est réveillé et/ou synchronisé. Le module à transpondeur 1 comprend comme capteur de mouvement au moins un gyromètre ou gyroscope 7. De préférence, le gyroscope 7 permet de déterminer une vitesse de rotation et un angle de rotation de la partie haute du corps de l'athlète pour déterminer un angle α de la partie haute du corps par exemple par rapport à une direction verticale ou perpendiculaire à la piste. Cette détermination du gyroscope n'est évidemment possible, que si l'orientation initiale est connue. Les signaux de mesure sont fournis directement à l'unité de traitement 4. L'angle d'inclinaison α peut aussi être déterminé par rapport à une direction horizontale.

Il est à noter que le capteur de mouvement du module à transpondeur 1 peut être également un ensemble, qui comprend un accéléromètre triaxial 8, un gyroscope triaxial 7 et un capteur magnétique triaxial non représenté. Cet ensemble définit un capteur inertiel à 9 axes.

Le module à transpondeur 1 peut encore comprendre un autre capteur de mouvement, qui est un accéléromètre 8 lié aussi à l'unité de traitement 4. Cet accéléromètre 8 peut mesurer l'accélération d'un athlète au moment du départ de la course et des cadences de foulées durant toute la course et jusqu'à l'arrivée. De plus, le gyroscope 7 peut être triaxial, ainsi que l'accéléromètre 8 de manière à opérer des mesures dans les trois directions x, y, z.

Dans le module à transpondeur 1, les signaux de mesure du gyroscope 7 et de l'accéléromètre 8 ou d'autres types de capteurs sont échantillonnés par l'unité de traitement 4 ou directement par le capteur lui-même avant une transmission à l'unité de traitement 4. Les signaux de mesure peuvent être transmis directement à la station de base 10 en utilisant l'unité de transmission sans fil 5. Toutefois, les signaux de mesure peuvent être améliorés notamment après filtrage et ensuite mémorisés et/ou envoyés par la suite à la station de base 10. Il peut aussi être prévu de traiter les données des différents capteurs et tout événement de détection. Il peut encore être prévu de traiter des caractéristiques de mouvement extraites, telles que la fréquence des pas et transmettre cette information à la station de base 10 en plus des données proprement dites de l'accéléromètre 8 et du gyroscope 7.

Il est encore à noter que les signaux reçus par l'antenne 2 liée à l'unité de réception 3 peuvent être des signaux à basse fréquence de l'ordre de 125 kHz, alors que les signaux transmis par l'antenne 6 liée à l'unité de transmission 5 peuvent être des signaux à fréquence relativement élevée, par exemple des signaux HF ou UHF à fréquence supérieure à 300 MHz. Toutefois, il peut être concevable d'avoir un module à transpondeur 1 avec une seule antenne de réception et d'émission commutable pour la réception ou l'émission de signaux de données. Dans ce cas de figure, il est préféré avoir une réception d'au moins un signal de réveil et une émission de signaux de données à une fréquence porteuse similaire avec une modulation des données transmises FSK, BPSK, QPSK ou ON-OFF Keying.

Comme il est possible de le visionner aux figures 2a à 2c, un athlète 20 peut passer la ligne d'arrivée 30 en position droite, c'est-à-dire avec un faible angle α d'inclinaison de la partie haute du corps par rapport à la verticale. Le module à transpondeur 1 passe donc en même temps que la partie haute du corps comme montré à la figure 2a. Si un athlète 20 se plie vers l'avant, lorsqu'il passe la ligne d'arrivée 30, le module à transpondeur 1 est encore en retrait d'une distance Δx de la ligne d'arrivée 30 comme montré à la figure 2b. Finalement lorsque le module à transpondeur 1 passe sur la ligne d'arrivée comme montré à la figure 2c et est détecté par la station de base du système de mesure de chronométrage, il obtient une estampille temporelle. Cette estampille temporelle est Δx/v trop tard en comparaison du temps officiel final de l'athlète 20, où v est la vitesse de l'athlète. Par exemple pour une course de sprint sur 100 m, la vitesse de l'athlète 20 est voisine de 12 m/s en passant la ligne d'arrivée 30.

En ayant une estimation de l'inclinaison α de la partie haute du corps de l'athlète 20, la vitesse v et la position moyenne du module à transpondeur 1 sur la partie haute du corps, il est possible de fournir par approximation l'erreur de détection et la corriger. A titre d'exemple, si le module à transpondeur 1 est placé à une distance d, par exemple à 25 cm en dessous des épaules de l'athlète 20, il peut être mesuré l'inclinaison α par le gyroscope du module à transpondeur 1 de la partie haute du corps d'un angle α de 66° comme montré aux figures 2b et 2c, avec une vitesse d'une course de sprint à 12 m/s. Cette vitesse de course est calculée dans le module à transpondeur 1 au moment du passage de la ligne d'arrivée 30 par les mesures des capteurs de mouvement 7, 8.

La distance d peut aussi être estimée sur la base de paramètres anthropométriques de chaque athlète. Cette distance d peut ainsi être une fonction de la hauteur de l'athlète. La distance d estimée peut aussi être directement fournie avec le fabricant de chaque dossard de course. Si l'angle d'inclinaison α est déterminé par rapport à une direction verticale, l'erreur sur la distance à l'arrivée est Δx = d·sin(α). Par contre, si l'angle d'inclinaison α est déterminé par rapport à une direction horizontale, l'erreur sur la distance à l'arrivée est Δx = d·cos(α).

Le temps de course pris par le module à transpondeur 1 d'un athlète peut être égal à 9.598 s. L'erreur sur la distance devient Δx = (25 cm)·sin(α) = 22.8 cm et l'erreur temporelle Δt = Δx/v = 0.019 s. Ainsi le temps d'arrivée correct serait alors à 9.579 s, qui est arrondi selon les règles de l'athlétisme à 9.58 s. Cela correspond au temps officiel de course à communiquer par exemple aux spectateurs de ladite course de manière plus rapide que la détermination correcte par l'image photofinish du temps officiel de course de chaque athlète 20.

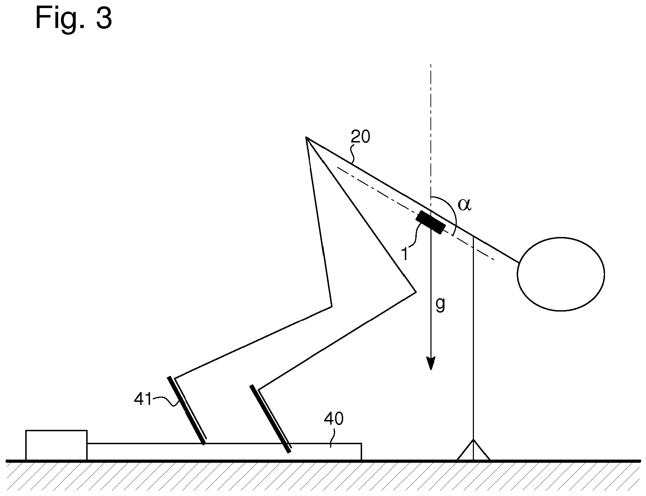

Comme représenté à la figure 3 au moment du départ, un athlète 20, qui est muni d'un module à transpondeur 1, est accroupi et en appui par ses deux pieds contre deux plots d'appui 41 d'un starting-block 40, qui est disposé et fixé sur le sol de la piste. De préférence, le capteur de mouvement peut être un capteur inertiel à 9 axes. Pour faire une estimation de l'inclinaison α de la partie haute du corps sur la base des signaux du gyroscope, il est nécessaire de connaître l'angle initial par exemple au début de la course. Cette angle α peut être obtenu en mesurant le vecteur du centre de gravité g par l'intermédiaire d'un accéléromètre triaxial. Cet angle initial est mémorisé dans le module à transpondeur, qui doit être activé pour cette mesure au moins juste avant le départ. L'angle initial est donc mémorisé durant la phase après l'annonce de préparation au départ et avant le coup de pistolet de départ de la course. Une fois que l'angle initial est connu, l'inclinaison de la partie haute du corps peut être calculée par des opérations de rotation sur la base des signaux du gyroscope durant la course et principalement à l'arrivée.

Il est à noter qu'il est nécessaire d'avoir une condition initiale pour permettre de déterminer l'angle d'inclinaison a. Le module à transpondeur avec le capteur de mouvement doit être calibré pour pouvoir effectuer cette mesure de l'angle d'inclinaison lors du passage de la ligne d'arrivée. Cette condition initiale peut être définie de préférence avant le départ de la course, quand l'athlète reste prêt comme montré à la figure 3, mais peut aussi être estimée durant la course avant le passage de la ligne d'arrivée.

Pour le procédé de mesure d'un temps de course pour la mise en action du système de mesure, il peut être prévu de mesurer l'angle de la partie haute du corps d'un athlète en course dans le module à transpondeur. Ce module peut être activé dès le départ ou dans des positions intermédiaires entre le départ et la ligne d'arrivée, ou à l'arrivée. De préférence le module à transpondeur est activé dès le départ de la course. Le capteur de mouvement, qui comprend au moins le gyroscope, est prévu pour déterminer un angle d'inclinaison de la partie haute du corps de l'athlète, qui porte le module à transpondeur. Une orientation initiale du module à transpondeur peut être définie et mémorisée juste avant le départ de la course. Ainsi une fois que le module à transpondeur s'approche de la ligne d'arrivée et par exemple en détectant des signaux d'interrogation à basse fréquence d'une antenne d'arrivée, une détermination de l'angle d'inclinaison et l'écart temporel sont calculés directement dans ledit module à transpondeur. Une fois calculés, il y a une transmission de signaux de données ou commandes à destination de la station de base pour définir un temps de course corrigé.

Il est à noter que, dans un mode de réalisation non couvert par les revendications, le module à transpondeur activé peut également transmettre toutes les données de mesure effectuées par ses capteurs à la station de base, qui dans ce cas détermine et calcule l'écart temporel et la correction du temps non officiel de chaque athlète.

A partir de la description qui vient d'être faite, plusieurs variantes du système et procédé de mesure avec correction du temps d'arrivée d'un athlète dans une course peuvent être conçues par l'homme du métier sans sortir du cadre de l'invention définie par les revendications. Il peut être prévu d'avoir plusieurs modules à transpondeur sur une partie du corps de l'athlète pour une détermination de l'angle d'inclinaison au passage de la ligne d'arrivée et la correction du temps d'arrivée non officiel.

## Revendications

1. Système de mesure avec correction d'un temps d'arrivée d'au moins un athlète (20) dans une course à pieds, le système de mesure comprenant au moins un module à transpondeur (1) personnalisé et placé sur une partie haute du corps d'un athlète (20) et une station de base (10), ladite station de base (10) étant configurée pour détecter le temps d'arrivée correspondant au temps de passage sur la ligne d'arrivée du module à transpondeur (1), ledit module à transpondeur (1) comprenant une unité de traitement (4) de données, de mesures ou de commandes, une unité de transmission (5) de signaux de données et/ou de mesures et/ou de commandes, et au moins un deux capteurs de mouvement (7, 8) pour fournir des signaux de mesure à l'unité de traitement (4),
**caractérisé en ce que** les capteurs de mouvement sont au moins un gyroscope (7) et un accéléromètre (8), qui sont reliés à l'unité de traitement (4) dans le module à transpondeur (1),
**en ce que** le gyroscope (7) est configuré pour déterminer un angle d'inclinaison α du corps de l'athlète par rapport à une direction verticale ou horizontale lors du passage de la ligne d'arrivée (30) pour une correction d'un temps d'arrivée de course,
**en ce que** le module à transpondeur (1) est configuré pour calculer la vitesse de course de l'athlète au moment du passage de la ligne d'arrivée (30) grâce aux mesures des capteurs de mouvement (7, 8),
**en ce que** l'unité de traitement (4) est configurée pour calculer une erreur temporelle Δt d'un temps de course de l'athlète (20) sur la base de l'angle d'inclinaison α par rapport à une direction verticale ou horizontale, de la position d du module à transpondeur (1) par rapport aux épaules de l'athlète et de la vitesse de l'athlète v à l'arrivée, l'erreur temporelle étant Δt = Δx/v, où Δx = d·sin(α) pour un angle d'inclinaison α selon une direction verticale ou Δx = d·cos(α) pour un angle d'inclinaison α selon une direction horizontale,
**en ce que** le module à transpondeur (1) est configuré pour transmettre l'erreur temporelle calculée via l'unité de transmission (5) à la station de base (10), et
**en ce que** la station de base (10) est configurée pour corriger le temps d'arrivée détecté avec l'erreur temporelle reçue.

2. Système de mesure selon la revendication 1, **caractérisé en ce que** le gyroscope (7) est un gyroscope triaxial, et **en ce que** l'accéléromètre (8) est un accéléromètre triaxial.

3. Système de mesure selon la revendication 1, **caractérisé en ce que** les capteurs de mouvement sont un ensemble de capteurs, défini en tant que capteur inertiel à 9 axes, qui comprend un accéléromètre triaxial (8), un gyroscope triaxial (7) et un capteur magnétique triaxial.

4. Système de mesure selon la revendication 1, **caractérisé en ce que** le gyroscope (7) est configuré pour être calibré au début d'une course.

5. Système de mesure selon la revendication 1, **caractérisé en ce que** l'unité de traitement (4) détermine un temps de course de l'athlète corrigé dans le module à transpondeur (1) pour une transmission du temps de course correct de l'athlète à la station de base (10).

6. Système de mesure selon l'une des revendications précédentes, **caractérisé en ce que** le module à transpondeur comprend en outre une unité de réception sans fil de signaux (3) pour recevoir par une antenne (2) des signaux de données ou commandes provenant de la station de base (10) ou d'un émetteur disposé au départ de la course ou le long d'un tracé de course.

7. Procédé de mesure avec correction du temps d'arrivée d'un athlète dans une course à pieds comprenant un système de mesure selon l'une des revendications précédentes, pour lequel le système de mesure comprend au moins un module à transpondeur (1) personnalisé et placé sur une partie haute du corps d'un athlète (20) et une station de base (10), ladite station de base (10) étant configurée pour détecter le temps d'arrivée correspondant au temps de passage sur la ligne d'arrivée du module à transpondeur (1), ledit module à transpondeur (1) comprenant une unité de traitement (4) de données, de mesures ou de commandes, une unité de transmission (5) de signaux de données et/ou de mesures et/ou de commandes, et au moins deux capteurs de mouvement (7, 8), qui sont au moins un gyroscope (7) et un accéléromètre (8) reliés à l'unité de traitement (4) dans le module à_transpondeur (1) pour fournir des signaux de mesure à l'unité de traitement (4), **caractérisé en ce que** le procédé comprend les étapes de :
- mesurer un angle d'inclinaison α de l'athlète (20) au moyen du gyroscope (7) du module à transpondeur (1) activé au moment de passer la ligne d'arrivée (30),
- calculer par le module à transpondeur (1) la vitesse de course de l'athlète au moment du passage de la ligne d'arrivée (30) grâce aux mesures des capteurs de mouvement (7, 8),
- effectuer un calcul d'une erreur temporelle Δt d'un temps d'arrivée de course de l'athlète (20) dans l'unité de traitement (4) du module à transpondeur sur la base de l'angle d'inclinaison α par rapport à une direction verticale ou horizontale, de la position d du module à transpondeur (1) par rapport aux épaules de l'athlète et de la vitesse de l'athlète v à l'arrivée, l'erreur temporelle étant Δt = Δx/v, où Δx = d·sin(α) pour un angle d'inclinaison α selon une direction verticale ou Δx = d·cos(α) pour un angle d'inclinaison α selon une direction horizontale,
- transmettre l'erreur temporelle calculée via l'unité de transmission (5) à la station de base (10), et
- corriger le temps d'arrivée détecté par la station de base (10) avec l'erreur temporelle reçue.

8. Procédé de mesure selon la revendication 7, **caractérisé en ce qu'**une fois activé, une mesure d'accélération de l'athlète est effectuée par un autre capteur de mouvement, qui est un accéléromètre (8) du module à transpondeur (1) de manière à détecter une variation de mouvement dudit module ou d'un niveau de vibrations dudit module.

9. Procédé de mesure selon l'une des revendications 7 et 8, **caractérisé en ce que** le module à transpondeur (1) est synchronisé dès le départ de la course ou en différents points de la course ou à proximité de la ligne d'arrivée par un signal d'interrogation ou de réveil.

10. Procédé de mesure selon la revendication 9, **caractérisé en ce qu'**une fois activé et synchronisé, le module à transpondeur (1) détermine un temps de course corrigé au passage de la ligne d'arrivée sur la base de mesures d'intensités d'un signal de champ magnétique d'une antenne à proximité de la ligne d'arrivée (30) et capté par une unité de réception (3), et **en ce que** le temps d'arrivée de course corrigé est transmis du module à transpondeur (1) à la station de base (10).

## Patentansprüche

1. Messsystem mit Korrektur einer Ankunftszeit mindestens eines Athleten (20) bei einem Wettlauf, wobei das Messsystem mindestens ein personalisiertes Transpondermodul (1) umfasst, das auf einem oberen Teil des Körpers eines Athleten (20) platziert ist, und eine Basisstation (10), wobei die Basisstation (10) ausgelegt ist, um die Ankunftszeit zu ermitteln, die der Zeit des Passierens der Ziellinie des Transpondermoduls (1) entspricht, wobei das Transpondermodul (1) eine Verarbeitungseinheit (4) von Daten, von Messungen oder von Steuerungen, eine Übertragungseinheit (5) von Signalen von Daten und/oder von Messungen und/oder von Steuerungen und mindestens zwei Bewegungssensoren (7, 8) umfasst, um Messsignale an die Verarbeitungseinheit (4) zu liefern,
**dadurch gekennzeichnet, dass** die Bewegungssensoren mindestens ein Gyroskop (7) und ein Beschleunigungsmesser (8) sind, die mit der Verarbeitungseinheit (4) im Transpondermodul (1) verbunden sind,
und dass das Gyroskop (7) ausgelegt ist, um einen Neigungswinkel α des Körpers des Athleten in Bezug auf eine vertikale oder horizontale Richtung beim Passieren der Ziellinie (30) für eine Korrektur einer Laufankunftszeit zu bestimmen, und
dass das Transpondermodul (1) ausgelegt ist, um die Laufgeschwindigkeit des Athleten im Augenblick des Passierens der Ziellinie (30) dank der Messungen der Bewegungssensoren (7, 8) zu berechnen,
und dass die Verarbeitungseinheit (4) ausgelegt ist, um einen Zeitfehler Δt einer Laufzeit des Athleten (20) auf der Basis des Neigungswinkels α in Bezug auf eine vertikale oder horizontale Richtung, der Position d des Transpondermoduls (1) in Bezug auf die Schultern des Athleten und der Geschwindigkeit des Athleten v bei Ankunft zu berechnen, wobei der Zeitfehler Δt = Δx/v ist, wobei Δx = d·sin(α) für einen Neigungswinkel α gemäß einer vertikalen Richtung oder Δx = d cos(a) für einen Neigungswinkel α gemäß einer horizontalen Richtung ist,
dass das Transpondermodul (1) ausgelegt ist, um den berechneten Zeitfehler über die Übertragungseinheit (5) an die Basisstation (10) zu übertragen, und
dass die Basisstation (10) ausgelegt ist, um die ermittelte Ankunftszeit mit dem empfangenen Zeitfehler zu korrigieren.

2. Messsystem nach Anspruch 1, **dadurch gekennzeichnet, dass** das Gyroskop (7) ein dreiachsiges Gyroskop ist und dass der Beschleunigungsmesser (8) ein dreiachsiger Beschleunigungsmesser ist.

3. Messsystem nach Anspruch 1, **dadurch gekennzeichnet, dass** die Bewegungssensoren eine Anordnung von Sensoren sind, definiert als 9-achsiger Trägheitssensor, der einen dreiachsigen Beschleunigungsmesser (8), ein dreiachsiges Gyroskop (7) und einen dreiachsigen Magnetsensor umfasst.

4. Messsystem nach Anspruch 1, **dadurch gekennzeichnet, dass** das Gyroskop (7) ausgelegt ist, um zu Beginn eines Laufs kalibriert zu sein.

5. Messsystem nach Anspruch 1, **dadurch gekennzeichnet, dass** die Verarbeitungseinheit (4) eine korrigierte Laufzeit des Athleten im Transpondermodul (1) für eine Übertragung der korrekten Laufzeit des Athleten an die Basisstation (10) bestimmt.

6. Messsystem nach einem der vorangehenden Ansprüche, **dadurch gekennzeichnet, dass** das Transpondermodul ferner eine drahtlose Signalempfangseinheit (3) umfasst, um über eine Antenne (2) Signale von Daten oder Steuerungen von der Basisstation (10) oder einem Sender zu empfangen, der am Start des Laufs oder entlang einer Laufstrecke angeordnet ist.

7. Messverfahren mit Korrektur der Ankunftszeit eines Athleten bei einem Wettlauf, umfassend ein Messsystem nach einem der vorangehenden Ansprüche, wobei das Messsystem mindestens ein personalisiertes Transpondermodul (1), das auf einem oberen Teil des Körpers eines Athleten (20) platziert ist, und eine Basisstation (10) umfasst, wobei die Basisstation (10) ausgelegt ist, um die Ankunftszeit zu ermitteln, die der Zeit des Passierens der Ziellinie des Transpondermoduls (1) entspricht, wobei das Transpondermodul (1) eine Verarbeitungseinheit (4) von Daten, von Messungen oder von Steuerungen, eine Übertragungseinheit (5) von Signalen von Daten und/oder von Messungen und/oder von Steuerungen und mindestens zwei Bewegungssensoren (7, 8) umfasst, die mindestens ein Gyroskop (7) und ein Beschleunigungsmesser (8) sind, die mit der Verarbeitungseinheit (4) im Transpondermodul (1) verbunden sind, um Messsignale an die Verarbeitungseinheit (4) zu liefern, **dadurch gekennzeichnet, dass** das Verfahren die folgenden Schritte umfasst:
- Messen eines Neigungswinkels α des Athleten (20) mittels des Gyroskops (7) des Transpondermoduls (1), das im Augenblick des Passierens der Ziellinie (30) aktiviert ist,
- Berechnen, durch das Transpondermodul (1), der Laufgeschwindigkeit des Athleten im Augenblick des Passierens der Ziellinie (30) dank der Messungen der Bewegungssensoren (7, 8),
- Durchführen einer Berechnung eines Zeitfehlers Δt einer Laufankunftszeit des Athleten (20) in der Verarbeitungseinheit (4) des Transpondermoduls auf der Basis des Neigungswinkels α in Bezug auf eine vertikale oder horizontale Richtung, der Position d des Transpondermoduls (1) in Bezug auf die Schultern des Athleten und der Geschwindigkeit des Athleten v bei Ankunft, wobei der Zeitfehler Δt = Δx/v ist, wobei Δx = d·sin(α) für einen Neigungswinkel α gemäß einer vertikalen Richtung oder Δx = d·cos(α) für einen Neigungswinkel α gemäß einer horizontalen Richtung ist,
- Übertragen des berechneten Zeitfehlers über die Übertragungseinheit (5) an die Basisstation (10), und
- Korrigieren der von der Basisstation (10) ermittelten Ankunftszeit mit dem empfangenen Zeitfehler.

8. Messverfahren nach Anspruch 7, **dadurch gekennzeichnet, dass**, sobald aktiviert, eine Messung der Beschleunigung des Athleten von einem anderen Bewegungssensor, der ein Beschleunigungsmesser (8) des Transpondermoduls (1) ist, derart durchgeführt wird, dass eine Schwankung der Bewegung des Moduls oder eines Vibrationsniveaus des Moduls ermittelt wird.

9. Messverfahren nach einem der Ansprüche 7 und 8, **dadurch gekennzeichnet, dass** das Transpondermodul (1) ab dem Start des Laufs oder an verschiedenen Punkten des Laufs oder in der Nähe der Ziellinie von einem Abfrage- oder Wecksignal synchronisiert wird.

10. Messverfahren nach Anspruch 9, **dadurch gekennzeichnet, dass**, sobald aktiviert und synchronisiert, das Transpondermodul (1) eine korrigierte Laufzeit beim Passieren der Ziellinie auf der Basis von Stärkemessungen eines Magnetfeldsignals einer Antenne in der Nähe der Ziellinie (30), das von einer Empfangseinheit (3) aufgefangen wird, bestimmt, und dass die korrigierte Laufankunftszeit vom Transpondermodul (1) an die Basisstation (10) übertragen wird.

## Claims

1. System for measuring and correcting a finish time of at least one athlete (20) in a running race, the measuring system comprising at least one transponder module (1) which is personalised and placed on an upper part of the body of an athlete (20) and a base station (10), said base station (10) being configured to detect the finish time corresponding to the crossing time on the finish line of the transponder module (1), said transponder module (1) comprising a unit (4) for processing data, measurements or commands, a unit (5) for transmitting data and/or measurement and/or command signals, and at least two motion sensors (7, 8) for providing measurement signals to the processing unit (4),
**characterised in that** the motion sensors are at least one gyroscope (7) and one accelerometer (8), which are connected to the processing unit (4) in the transponder module (1),
**in that** the gyroscope (7) is configured to determine an angle of inclination α of the body of the athlete with respect to a vertical or horizontal direction upon crossing the finish line (30) for a correction of a race finish time,
**in that** the transponder module (1) is configured to calculate the running speed of the athlete when crossing the finish line (30) using measurements from the motion sensors (7, 8),
**in that** the processing unit (4) is configured to calculate a time error Δt for a race time of the athlete (20) based on the angle of inclination α with respect to a vertical or horizontal direction, of the position d of the transponder module (1) with respect to the shoulders of the athlete and the speed v of the athlete at the finish line, the time error being Δt = Δx/v, where Δx = d·sin(α) for an angle of inclination α in a vertical direction or Δx = d·cos(α) for an angle of inclination α in a horizontal direction.
**in that** the transponder module (1) is configured to transmit the calculated time error via the transmission unit (5) to the base station (10), and
**in that** the base station (10) is configured to correct the detected finish time with the received time error.

2. Measuring system according to claim 1, **characterised in that** the gyroscope (7) is a three-axis gyroscope, and **in that** the accelerometer (8) is a three-axis accelerometer.

3. Measuring system according to claim 1, **characterised in that** the motion sensors are an assembly of sensors, defined as a 9-axis inertial sensor, which comprises a three-axis accelerometer (8), a three-axis gyroscope (7) and a three-axis magnetic sensor.

4. Measuring system according to claim 1, **characterised in that** the gyroscope (7) is configured to be calibrated at the start of a race.

5. Measuring system according to claim 1, **characterised in that** the processing unit (4) determines a race time for the athlete corrected in the transponder module (1) for transmission of the correct race time for the athlete to the base station (10).

6. Measuring system according to any of the preceding claims, **characterised in that** the transponder module further comprises a wireless signal receiver unit (3) for receiving via an antenna (2) data or command signals from the base station (10) or from a transmitter arranged at the start of the race or along the race course.

7. Method for measuring with correction the finish time of an athlete in a running race comprising a measuring system according to any of the preceding claims, wherein the measuring system comprises at least one personalised transponder module (1) placed on an upper part of the body of an athlete (20) and a base station (10), said base station (10) being configured to detect the finish time corresponding to the crossing time on the finish line of the transponder module (1), said transponder module (1) comprising a unit (4) for processing data, measurements or commands, a unit (5) for transmitting data and/or measurement and/or command signals, and at least two motion sensors (7, 8), which are at least one gyroscope (7) and one accelerometer (8) connected to the processing unit (4) in the transponder module (1) for providing measurement signals to the processing unit (4), **characterised in that** the method comprises the steps of:
- measuring an angle of inclination α of the athlete (20) by means of the gyroscope (7) of the transponder module (1) activated at the moment of crossing the finish line (30),
- calculating by the transponder module (1) the running speed of the athlete at the moment of crossing the finish line (30) using measurements from the motion sensors (7, 8),
- performing a calculation of a time error Δt of a race finish time for the athlete (20) in the processing unit (4) of the transponder module based on the angle of inclination α with respect to a vertical or horizontal direction, of the position d of the transponder module (1) with respect to the shoulders of the athlete and the speed v of the athlete at the finish line, the time error being Δt = Δx/v, where Δx = d·sin(α) for an angle of inclination α in a vertical direction or Δx = d·cos(α) for an angle of inclination α in a horizontal direction.
- transmitting the calculated time error via the transmission unit (5) to the base station (10), and
- correcting the detected finish time by the base station (10) with the received time error.

8. Measuring method according to claim 7, **characterised in that**, once activated, an acceleration measurement of the athlete is performed by another motion sensor, which is an accelerometer (8) of the transponder module (1) in order to detect a variation in motion of said module or a vibration level of said module.

9. Measuring method according to any of claims 7 and 8, **characterised in that** the transponder module (1) is synchronised at the start of the race or at different points on the course or close to the finish line by an interrogation or wake-up signal.

10. Measuring method according to claim 9, **characterised in that** once activated and synchronised, the transponder module (1) determines a corrected race time on the crossing of the finish line based on intensity measurements of a magnetic field signal from an antenna close to the finish line (30) and captured by a receiver unit (3), and **in that** the corrected race finish time is transmitted from the transponder module (1) to the base station (10).
